# EUROPEAN PATENT SPECIFICATION

(11) **EP 1 845 805 B1**
(45) Date of publication and mention of the grant of the patent: **07.11.2012**
(21) Application number: 05772935.2
(22) Date of filing: 08.02.2005
(51) Int. Cl.: A23L 2/00, A61K 33/00, A23L 2/38

(54) **NON-ALCOHOLIC BEVERAGE ENRICHED WITH 1H2 16O**
MIT 1H2 16O VERSETZTER SOFTDRINK
BOISSON SANS ALCOOL ENRICHIE DE 1H2 16O

(43) Date of publication of application: 24.10.2007
(73) Proprietor: Woodford Associates Limited, London W4 3HR (GB); Soloviev, Sergey Pavlovich, Moscow 115432 (RU)
(72) Inventor: SOLOVIEV, Sergej Pavlovich, Moscow, 115432 (RU)
(74) Representative: Hayes, Adrian Chetwynd
(86) International application number: PCT/RU2005/000045
(87) International publication number: WO 2006/085785

(56) References cited:
- WO-A-03/049748
- WO-A-2005/070438
- US-A- 5 855 921
- PATENT ABSTRACTS OF JAPAN vol. 2003, no. 12, 5 December 2003 (2003-12-05) & JP 2004 315487 A (INTERNATL SCIENT:KK), 11 November 2004 (2004-11-11)
- PATENT ABSTRACTS OF JAPAN vol. 2003, no. 12, 5 December 2003 (2003-12-05) & JP 2004 075654 A (INTERNATL SCIENT:KK), 11 March 2004 (2004-03-11)
- SOMLYAI G ET AL: "THE BIOLOGICAL EFFECTS OF DEUTERIUM-DEPLETED WATER, A POSSIBLE NEW TOOL IN CANCER THERAPY" DEUTSCHE ZEITSCHRIFT FUER ONKOLOGIE, HEIDELBERG, DE, vol. 30, no. 4, 1998, pages 91-94, XP009006973 ISSN: 0931-0037
- BILD W ET AL: "RESEARCH CONCERNING THE RADIOPROTECTIVE AND IMMUNOSTIMULATING EFFECTS OF DEUTERIUM-DEPLETED WATER" REVUE ROUMAINE DE PHYSIOLOGIE, EDITIONS DE L'ACADEMIE DE LA REPUBLIQUE SOCIALISTE DE LA ROUMA, RO, vol. 36, no. 3/4, July 1999 (1999-07), pages 205-218, XP001145877 ISSN: 0035-399X

## Description

### Technical Field

The present invention relates to non-alcoholic beverage production. More specifically, this invention relates to production of non-alcoholic beverage enriched with ¹H₂¹⁶O, in other words non-alcoholic beverage with increased ¹H₂¹⁶O content in non-alcoholic beverage composition in comparison with typical non-alcoholic beverage composition.

### Background of the Invention

The quality and purity of drinking water and water as a component in the composition of non-alcoholic beverages are one of determinative factors for life quality and human health.

Water as a chemical agent is a substance consisting of water molecules. However there is no absolutely pure natural water. The natural water always contains a quantity of different suspension particles, chemical and biological admixtures. I.e. any natural water (including any drinking water) is a composition of water as a chemical agent and some other substances.

The purification of water is a vital necessity today. Water purification methods depend on subsequent use of water and can be different, such as filtration, distillation, reverse osmosis and so on. The traditional water purification methods are able to eliminate only admixtures from water and have no effect on water as a chemical agent. I.e. they do not change the ratio between isotope varieties of water molecules.

The molecule of water H₂O consists of two chemical elements - hydrogen H and oxygen O. Either of two elements also consists of several isotopes.

Hereinafter:
the term «hydrogen» (lettering: H) means a chemical element as a total of stable nonradioactive hydrogen isotope varieties;
the term «oxygen» (lettering: O) means a chemical element as a total of stable nonradioactive oxygen isotope varieties;
the natural hydrogen consists of stable nonradioactive isotopes:
   - protium (lettering ¹H);
   - deuterium (lettering ²H, historical symbol D, further can be used lettering ²H or equivalent lettering D).
the natural oxygen consists of three stable nonradioactive isotopes:
   - oxygen-16 (lettering ¹⁶O),
   - oxygen-17 (lettering ¹⁷O),
   - oxygen-18 (lettering ¹⁸O),
(the present invention concerns the said stable nonradioactive isotopes only);

Any water as chemical agent is a composition of 9 isotope varieties of water molecule such as: ¹H₂¹⁶O, ¹H¹⁷O ¹H₂¹⁸O, ¹H²H¹⁶O, ¹H²H¹⁷O, ¹H²H¹⁸O, ²H₂¹⁶O, ²H₂¹⁷O, ²H₂¹⁸O formed by stable isotopes of hydrogen - ¹H, ²H, and stable isotopes of oxygen - ¹⁶O, ¹⁷O, ¹⁸O. The other name for these isotope varieties of water molecule is isotopologues.

The term «isotopologue» is defined in accordance with IUPAC Compendium of Chemical Terminology 2nd Edition (1997) and refers to a molecular entity that differs only in isotopic composition (number of isotopic substitutions), e.g. ¹H₂¹⁶O, ¹H²H¹⁶O, ¹H₂¹⁸O. Herein and after the terms «isotope variety of water molecule» and «isotopologue» are used as convertible terms.

The content of water isotopologues in Ocean Water is stated as the internationally accepted water standard VSMOW. In Ocean Water the level of ¹H₂¹⁶O molecules comprising light isotopes ¹H and ¹⁶O is 99.731% (Vienna Standard Mean Ocean Water, VSMOW), and about 0.2683% of the Ocean Water is formed by water molecules comprising heavy isotopes ²H, ¹⁷O, ¹⁸O (0.0372% ¹H₂¹⁷O, 0.199983% ¹H₂¹⁸O, 0.031069% ¹H²H ¹⁶O, and etc.) (Rothman et al., J. Quant. Spectrosc. Radiat. Transfer, 1998, 60, 665. Rothman et al., J. Quant. Spectrosc. Radiat. Transfer, 2003, 82, p.9). The abundance of water isotopologues in natural water varies depending on Earth regions and climate conditions and is typically expressed as the deviation, δ, relative to the VSMOW standard. The natural water with maximum content of light water isotopologue ¹H₂¹⁶O was found in Antarctica (Standard Light Antarctic Precipitation, SLAP), wherein said δ-values of residual heavy isotopes are δ²H = -415.5960, δ¹⁷O = -28.1‰, and δ¹⁸O = -53.9‰ that corresponds to the 99.757 % level of light water isotopologue ¹H₂¹⁶O (R. van Trigt, Laser Spectrometry for Stable Isotope Analysis of Water Biomedical and Paleoclimatological Applications, 2002, Groningen: University Library Groningen, p. 50).

Thus, natural water with the abundance of light water isotopologue ¹H₂¹⁶O more than 99.757% is not found in nature.

In natural water the residual concentration of the molecules, comprising ²H, ¹⁷O, ¹⁸O heavy isotopes, such as ¹H¹⁷O, ¹H¹⁸O, ¹H²H¹⁶O ¹H²H¹⁷O, ¹H²H¹⁸O, ²H₂¹⁶O,²H₂¹⁷O, ²H₂¹⁸O can amount to 2.97 g/l.

Since total levels of deuterium-comprising isotopologues in water is rather more 0.031% (Rothman et al., J. Quant. Spectrosc. Radiat. Transfer, 1998, 60, 665. Rothman et al., J. Quant. Spectrosc. Radiat. Transfer, 2003, 82, p.9) a complete depletion of natural water of deuterium-comprising isotopologues provides water with the level of light water isotopologue ¹H₂¹⁶O no more than 99.76%.

Thus, water with abundance of light water isotopologue ¹H₂¹⁶O more than 99.76% is unknown from the art.

Method and apparatus for production of water with abundance of ¹H₂¹⁶O liglit water isotopologue more than 99.76% are also unknown from the art.

Light and heavy water isotopologues differ appreciably in their physical properties. For example, there are the distinctions between the boiling-points, freezing-points, densities. It is well-documented that light and heavy water isotopologues have distinct properties in biological system and distinctly affect properties of substances in solutions, for example, the rate of biochemical reactions; the enthalpy of association of several binding systems, including protein-carbohydrate, small molecule-small molecule, protein-peptide, and protein-nucleic acid; the thermodynamics (free-energy, enthalpy, entropy and heat-capacity changes) of a ligand binding; the enthalpy of protein unfolding; the thermodynamic stability and formation of functional structures of nucleic acids (Chervenak et al. JAGS, 1994, 116 (23): 10533-10539. Makhatadze et al., Nature Struct. Biol., 1995, 2 (10): 852-855. Connelly et al., PNAS, 1994, 91: 1964-1968. Cupane et al., Nucleic Acids Res. 1980, 8 (18): 4283-4303):

As mentioned above, in typical natural water the residual concentration of molecules, comprising ²H,¹⁷O, ¹⁸O heavy isotopes can amount to 2.97 g/l by weight In nature the less concentration of molecules, comprising ²H, ¹⁷O, ¹⁸O heavy isotopes, was found in Antarctica and corresponds to the 99.757 % level of light water isotopologue ¹H₂¹⁶O.

Heavy isotopes-comprising: molecules in mammal organism can lead to a change of normal biochemical processes and to a decrease of functional resources of the organism.

There is the barest necessity to increase the content of light water molecules of 1H₂¹⁶O and to decrease the content ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O molecules in human organism, as a result there is the improvement of human wellness and life quality.

The content of isotope varieties of water molecule such as:¹H₂¹⁶O, ¹H2¹⁷O, ¹H₂¹⁸O, ¹H²H¹⁶O, ¹H²H¹⁷O, ¹H²H¹⁸O ²H₂¹⁶O, ²H₂¹⁷O, ²H₂¹⁸O in human organism directly depend on the content of said isotope varieties of water molecule in a non-alcoholic beverage and drinking water used by human.

Non-alcoholic beverage on basis deuterium depleted water has known from prior art.

Document JP 2004-315487 A (INTERTL SCIENT:KK), 11.11.2004 discloses a beverage for increasing muscular build-up. The beverage uses deuterium depleted water (the content of deuterium is 140 ppm or less) as a raw material.

Document JP 2004075654 A (INTERTL SCIENT:KK), 11.03.2004 discloses a pharmaceutical formulation and a medicinal solution (i.e. a beverage) for the treatment of osteoporosis and prostatic hypertrophy. Deuterium is present in the water content thereof in an amount of from 0.1 ppm to 110 ppm.

Document W003049748 A (HYD KUHATO-FEJLESZT&ODBLAC; SOMLYAI GABOR), 19.06.2003 discloses pharmaceutical or foodstuff products (including beverages) for the treatment and curing of diabetes. The products comprise water with a deuterium content of 0.01-1335 ppm.

Document US5855921 A (SOMLYAI TABOR); 05.01.1999 discloses products for curing tumors diseases. The products comprise water or aqueous solutions as active agents. The products are suitable for human consumption and have a deuterium content of 0.1 to 110 ppm.
Documents SOMLYAI G ET AL: "BIOLOGICAL EFFECTS OF DEUTERIUM-DEPLETED WATER, A POSSIBLE NEW TOOL IN CANCER THERAPY" DEUTSCHE ZEITSCHRIFT FUER ONKOLOGIE, HEIDELBERG, DE, vol.30, no.4, 1998, pages 91-94 and BILD W ET AL "RESEARCH CONCERNING THE RADIOPROTECTIVE AND IMMUNOSTIMULATING EFFECTS OF DEUTERIUM-DEPLETED WATER" REVUE ROUMAINE DE PHISIOLOGIE, EDITIONS DE L'ACADEMIE DE LA REPUBLICQUE SICIALISTE DE LA ROUMA, RO, vol.36, no.3/4, 07.1999 pages 205-218, discuss the anti-tumor and immunostimulating effects of deuterium-depleted water.

None of the above prior art documents disclose that it is necessary to remove not only the deuterium, but also the heavy oxygen isotopologue content of a beverage. Nor do they disclose the control of the ¹H₂¹⁶O content. None of these prior art documents disclose that a beverage prepared with water enriched with ¹H₂¹⁶O has improved characteristics (taste, colour, smell) as a final beverage.

A non-alcoholic beverage with abundance of light water isotopologue ¹H₂¹⁶O more than 99.76% is unknown from the art.

A drinking water with abundance of light water isotopologue ¹H₂¹⁶O more than 99.76% is unknown from the art.

It is an object of the present inventlon to provide a non-alcoholic beverage comprising water with abundance of light water isotopologue ¹H₂¹⁶O more than 99.76%.

Herein and after, dririking water is one of the type of a non-alcoholic beverage.

### Brief Description of the Drawings

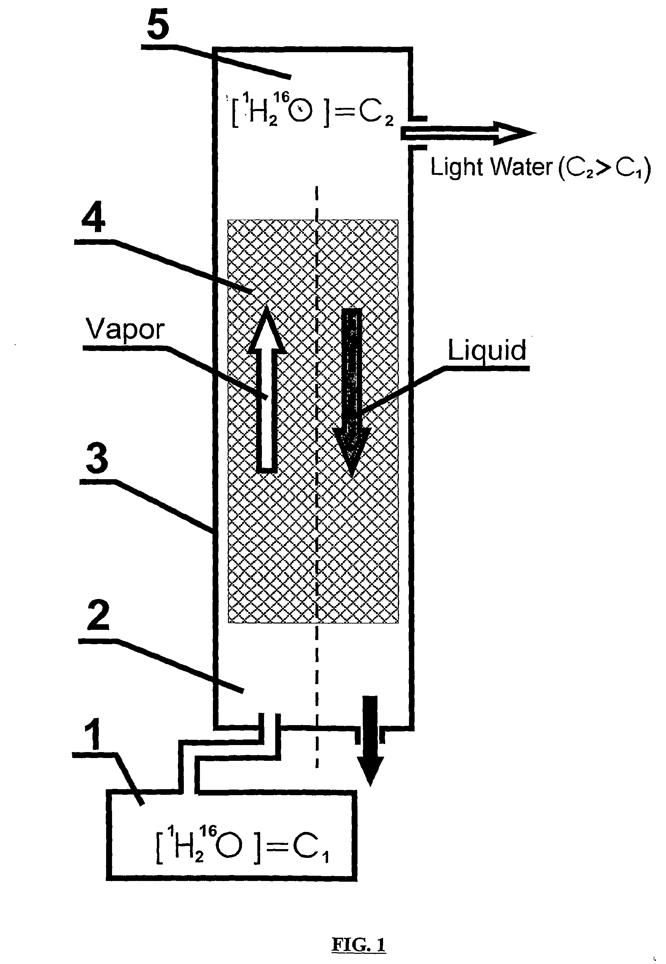

FIG. 1 is a schematic side view of an apparatus for the production of the water comprising from 99.76% to 99.99% of ¹H₂¹⁶O by weight.

### Disclosure of Invention

The present invention provides a non-alcoholic beverage enriched with ¹H₂¹⁶O, wherein the content of ¹H₂¹⁶O is no less than 99.76% by weight of water of said non-alcoliolic beverage, comprising:
A) a highly pure light water in an amount from 20% to 99:9% by weight of said non-alcoholic beverage, wherein the highly pure light water is a composition comprising from 99.76% to 99.99% of ¹H₂¹⁶O and residual amounts of ¹H₂¹⁷O, ¹H₂¹⁸O, ¹H²H¹⁶O,¹H²H¹⁷O, ¹H²H¹⁸O, ²H₂¹⁶O ²H₂¹⁷O, ²H₂¹⁸O up to 100% correspondingly,
B) a typical water with typical content of H²H₂¹⁶O in an amount from 0 % to 80% by weight of said non-alcoholic beverage, wherein said water is a composition comprising from 99:731 % to 99:757% of ¹H₂¹⁶O and residual amounts of ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O up to 100% correspondingly; and
C) a physiologically acceptable component in an amount up to 100% by weight of said non-alcoholic beverage.

Preferably, the present invention provides a non-alcoholic beverage enriched with ¹H₂¹⁶O, wherein the content of ¹H₂¹⁶O is no less than 99.80% by weight of water of said non-alcoholic beverage, comprising:
A) a highly pure light water in an amount from 20% to 99.9% by weight of said non-alcoholic beverage, wherein the highly pure light water is a composition comprising from 99.80 % to 99.99% of ¹H₂¹⁶O and residual amounts of ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O up to 100% correspondingly;
B) a typical water with typical content of ¹H₂¹⁶O in an amount from 0 % to 80% by weight of said non-alcoholic beverage, wherein said water is a composition comprising from 99.731 % to 99.757% of ¹H₂¹⁶O and residual amounts of ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O up to 100% correspondingly; and
C) a physiologically acceptable component in an amount up to 100% by weight of said non-alcoholic beverage.

Herein and after the definitions, «water with abundance of light water isotopologue ¹H₂¹⁶O more than 99.76%», «water with increased content ¹H₂¹⁶O» and «water enriched with ¹H₂¹⁶O» are used as convertible terms.

Herein and after, the term "highly pure light water" refers to water comprising from 99.76% to 99.99% of the most light isotope variety of water molecules, lettering ¹H₂¹⁶O.

Herein and after the term «typical water» means any water with content of ¹H₂¹⁶O within the limits of VSMOW-SLAP standards, i.e. from 99.731% to 99.757 % by weight of water.

Further, the present invention provides a non-alcoholic beverage enriched with ¹H₂¹⁶O, wherein the highly pure light water is selected from the group comprising distilled water, deionized water, reverse osmosis water.

Further, the present invention provides a non-alcoholic beverage enriched with ¹H₂¹⁶O, wherein the typical water with typical content of ¹H₂¹⁶O is selected from the group comprising drinking water, mineralized water, mineral water, distilled water, deionized water, reverse osmosis water.

Further, the present invention provides a non-alcoholic beverage enriched with ¹H₂¹⁶O, wherein the physiologically acceptable component is at least one selected from the group comprising natural fruit juice, vegetable juice, berry juice, nectar; dry beverage composition, dietary fiber, food component.

Further, the present invention provides a non-alcoholic beverage enriched with ¹H₂¹⁶O, wherein the physiologically acceptable component is at least one selected from the group comprising inorganic salt, mineral, nutrient, vitamin, flavanol, anti-oxidant, flavouring, extract, essence, colorant, aromatizator, food acid, bracer, technological additive,

Further, the present invention provides a non-alcoholic beverage enriched with ¹H₂¹⁶O, wherein said non-alcoholic beverage is a drinking water selected from the group comprising table drinking water, mineralized water, mineral water, mineral table water, treatment-prophylactic mineral water, mineral-medicinal water, baby drinking water and can be saturated with carbon dioxide.

Further, the present invention provides a non-alcoholic beverage enriched with ¹H₂¹⁶O, wherein said non-alcoholic beverage is a blended beverage selected from the group comprising table beverage, beverage for special purpose, refreshing beverage, cool beverage, tonic, lemonade, non-alcoholic cocktail and can be saturated with carbon dioxyde.

Further, the present invention provides the non-alcoholic beverage enriched with ¹H₂¹⁶O, wherein said non-alcoholic beverage is selected from the group comprising juice, nectar, kissel, mors, tea, kvass, non-alcoholic beer and can be saturated with carbon dioxide:

The present invention is directed to non-alcoholic beverage compositions comprising:
(A) a first component which is highly pure light water;
(B) a second component which is typical water; and
(C) a third component which is a physiologically acceptable component.

### First component (A) - highly pure light water

According to the present invention it is possible to produce water enriched with ¹H₂¹⁶O in an amount more than 99.76 % and up to 99.99% by weight of water. Water can be purified not only of typical chemicals, and admixtures, but also of molecules, such as: ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O, which can amount up to 2.97 g/l and are a kind of admixtures concerning main component of water, which is ¹H₂¹⁶O. As a result, the water becomes isotope homogenous substance consisting of ¹H₂¹⁶O in an amount up to 99.99%, in other words, light water. This light water is the pure water to a greater extent than any other purified water with typical isotope composition, it is highly pure light water. Thus, one can reach a qualitatively new and higher level of the water purity.

Thus, highly pure light water is a composition comprising from 99.76% to 99.99% of ¹H2¹⁶O and residual amounts of ¹H₂¹⁷O, ¹H₂¹⁸O, ¹H²H¹⁶O, ¹H²H¹⁷O, ¹H²H¹⁸O, ²H₂¹⁶O; ²H₂¹⁷O, ²H₂¹⁸ up to 100% correspondingly.

For practicing the invention we offer a method and apparatus for production of highly pure light water.

Highly pure light water comprising more than 99.76 % of light isotopologue ¹H₂¹⁶O is prepared by distillation of typical water with typical content ¹H₂¹⁶O with using the apparatus of FIG. 1. It is prepared by methods providing simultaneous depletion from typical water of 8 isotope varieties of water molecules comprising heavy isotopes ²H, ¹⁷O, and ¹⁸O such as: ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O.

The process of the distillation includes:
- evaporating natural water comprising [C₁] of light isotopologue ¹H₂¹⁶O in boiling (see FIG 1,1) to produce water vapor;
- supplying the water vapor to the bottom (2) of distillation column (3);
- carrying out vapor-liquid contact between a descending liquid and an ascending vapor mainly on the surface of the contact device (4) (e.g. structured or random packing) within the distillation column, simultaneously the liquid and the vapor flow in mutually opposite directions over the surface of the contact device along the main flow direction which is the direction of the column axis;

- condensing water vapor with concentration of light isotopologue ¹H₂¹⁶O [C₂] on condenser (5) installed on the top of the distillation column;
- collecting a part of condensate as condensed highly pure light water comprising the increased content (more than 99.76 %) of light isotopologue ¹H₂¹⁶O[C₂ > C₁].

After respective treatment one can get highly pure light water which is distilled water, deionized water, reverse osmosis water, ultra-pure water, etc. These types of water differ by concentration of chemical substances, but it always comprises more than 99.76% of light isotopologue ¹H₂¹⁶O.

There is a molecular spectroscopic method for direct determination of ¹H₂¹⁶O content in samples. (Rothman et al., J. Quant Spectrosc. Radiat. Transfer, 1998, 60, 665. Rothman et al., J. Quant. Spectrosc. Radiat. Transfer, 2003, 82, p.).

Then, the highly pure light water with increased content of light isotopologue ¹H₂¹⁶O is used for manufacturing of the drinking water and non-alcoholic beverage enriched with ¹H₂¹⁶O in an amount no less than 99.76 % by weight of water.

In the present invention highly pure light water can amount from 20% to 99.9% by weight of non-alcoholic beverage composition.

### Second component (B)-typical water.

Typical water is the .water with content of ¹H₂¹⁶O within the limits of VSMOW-SLAP standards, i.e. from 99.731% to 99.757 % by weight of water. In other words typical water is a composition comprising from 99.731 % to 99.757 % of ¹H₂¹⁶O and residual amounts of ¹H₂¹⁷O, ¹H₂¹⁸O, ¹H²H¹⁶O, ¹H²H¹⁷O, ¹H²H¹⁸O, ²H₂¹⁶O, ²H₂¹⁷O,²H₂¹⁸O up to 100% correspondingly. Thus dm³ of the water can include up to 2.97 g of the water molecules, comprising ²H, ¹⁷O,¹⁸O heavy isotopes.

This is a significant value, compared with the concentration of other typical components in natural water. For example, the total mineralization of water can amount from 0.2 to 1.0 g/l for table drinking water and more than 1.0 g/l for mineral Water.

Typical water can be drinking water, mineralized water, mineral water, distilled water, deionized water, reverse osmosis water, etc. These types of water differ by concentration of chemical substances. But these types of water always have typical content of ¹H₂¹⁶O from 99.731% to 99.757% by weight of water.

In the present invention typical water can amount from 0% to 80% by weight of non-alcoholic beverage composition.

### Third component (C) physiologically acceptable component

Examples of physiologically acceptable component include natural fruit juice, vegetable juice, berry juice, nectar, dry beverage composition, dietary fiber, food component, inorganic salt, mineral, nutrient, Vitamin, flavanol, anti-oxidant; flavouring, extract, essence, colorant, aromatizator, food acid, bracer, technological additive, etc. or mixture thereof.

Examples of natural fruit juice which can be used in the beverages of the present invention as a physiologically acceptable component, include apple juice, lemon juice, orange juice, grapefruit juice, etc. or mixture thereof.

Examples of natural vegetable juice which can be used in the beverages of the present invention as a physiologically acceptable component, include tomato juice, carrot juice, etc. or mixture thereof.

Examples of natural berry juice which can be used in the beverages of the present invention as a physiologically acceptable component, include black-currant juice, cranberries juice, etc. or mixture thereof.

Examples of nectar which can be used in the beverages of the present invention as a physiologically acceptable component, include peach nectar, banana nectar, etc. or mixture thereof.

Examples of dry beverage composition which can be used in the beverages of the present invention as a physiologically acceptable component, include orange-flavored dry beverage mix, powdered pink lemonade beverage mix, etc. or mixture thereof.

Examples of water soluble dietary fiber which can be used in the beverages of the present invention as a physiologically acceptable component, include pectin, gum, arabinogalactan, etc. or mixture thereof.

Food component used of the present invention as physiologically acceptable additive in non-alcoholic beverage composition means any food component usually used in ordinary non-alcoholic beverages in forms, which are necessary for this. Examples of alcoholic beverage of the present invention comprising food components include milk beverage, juice beverage, non-alcoholic cocktail, etc.

Food component used of the present invention as physiologically acceptable component in non-alcoholic beverage composition means any food component, for example, dairy produces, usually used in ordinary non-alcoholic beverages, in forms, which are necessary for this. Examples of non-alcoholic beverage of the present invention comprising food components include milk non-alcoholic cocktail, milk-fruit cocktail, etc.

Examples of food component, which can be used in the beverages of the present invention, include milk, concentrated low-fat milk, dried milk, cream, chocolate, egg, cocoa, juice, etc. or mixture thereof.

Examples of food component, which can be used in the beverages of the present invention, include milk, concentrated milk, concentrated low-fat milk, dried milk, cream, chocolate, egg, cocoa, natural juice, etc. or mixture thereof.

Examples of inorganic salts, which can be used in the beverages of the present invention as a physiologically acceptable component, include sodium chloride, sodium bicarbonate, calcium chloride, magnesium sulfate, etc. or mixture thereof.

Examples of minerals, which can be used in the beverages of the present invention as a physiologically acceptable component, include calcium, Magnesium, boron, chromium, cobalt, copper, fluoride, germanium, iodine, iron, lithium, magnesium, manganese, molybdenum, phosphorus, potassium, selenium, silicon, sodium, sulfur vanadium, zinc, etc. or mixture thereof.

Examples of nutrients, which can be used in the beverages of the present invention as a physiologically acceptable component; include carbohydrates, protein, lipid, essential fatty acid, amino acid and its derivation, etc. or mixture thereof.

Examples of amino acid and its derivation include phenylalanine, isoleucine, zinc amino acid chelate, etc. or mixture thereof.

Examples of vitamins, which can be used in the beverages of the present invention as a physiologically acceptable component, include retinol and retinoid, ascorbic acid, tocopherol, calciferol, thiamin, riboflavin, niacin, pantothenates, pyridoxine, folic acid, cobalamin, biotin, choline, inositol, lipoic acid, carnitine and their derivatives, etc. or mixture thereof.

Examples of synthetic or natural flavanol, which can be used in the beverages of the present invention as a physiologically acceptable component, include catechin, epicatechin, gallocatechin, epigallocatechin, epicatechin gallate, and epigallocatechin gallate and their derivatives, etc. or mixture thereof. The amount of flavanols in the compositions of the present invention can vary. However, preferably from 0.001% to 5%, of one or more flavanols is utilized, by weight of the composition.

Examples of antioxidant, which can be used in the beverages of the present invention as a physiologically acceptable component, include ascorbic acid, alpha-tocopherol, selenium.

The compositions of the present invention can contain an effective amount of one or more sweeteners as a physiologically acceptable component, including carbohydrate sweeteners and natural and/or artificial no/low calorie sweeteners.

Examples of sweetener include natural sweeteners, such as sucrose, fructose-glucose liquid sugar, purified honey, glycyrrhizin, stevioside, the protein sweetener thaumatin, the juice of Luo Han Guo; or artificial non-caloric sweeteners, such as aspartame, saccharin, cyclamates, acesulfame K, etc. or mixture thereof. Sweetened beverages will typically comprise from 0.1% to 20% natural sweetener. The total non-caloric sweetener is preferably utilized at levels from 0.0001% to 5% by weight of composition.

One or more flavoring agents as a physiologically acceptable component are recommended for the embodiments of me present invention in order to enhance their palatability. Any natural or synthetic flavor agent can be used in the present invention. For example, one or more botanical and/or fruit flavors may be utilized herein. As used herein, such flavors may be synthetic or natural flavors.

Examples of fruit flavourings include apple flavor, citrus flavor, grape flavor, raspberry flavor, cranberry flavor, cherry flavor, grapefruit flavor; and also exotic flavors such as, for example, passion fruit flavors, mango flavors, pineapple flavors, cupuacu flavors, guava flavors, cocoa flavors, papaya flavors, peach flavors, and apricot flavors, etc. or mixture thereof.

Examples of botanical flavors include tea, aloe vera, guarana, ginseng, ginkgo, hawthorn, hibiscus, rose hips, chamomile, peppermint, fennel, ginger, licorice, lotus seed, schizandra, saw palmetto, sarsaparilla, safflower, St. John's Wort, curcuma, cardimom, nutmeg, cassia bark, buchu, cinnamon, jasmine, haw, chrysanthernum, water chestnut, sugar cane, lychee, bamboo shoots, vanilla, coffee, etc. or mixture thereof. Typically the flavoring agents are conventionally available as concentrates or extracts or in the form of synthetically produced flavoring esters, alcohols, aldehydes, terpenes, sesquiterpenes, and the like.

Examples of herbal extracts, which can be used in the beverages of the present invention as a physiologically acceptable component, include guarana extract, aloe vera extract, ginkgo extract, korean ginseng extract, etc. or mixture thereof.

Examples of essence, which can be used in the beverages of the present invention as a physiologically acceptable component, include orange essence, blackcurrant essence, lemon essence, lime essence, cranberry essence, etc. or mixture thereof.

Examples of synthetic or natural coloring agent which can be used in the beverages of the present invention as a physiologically acceptable component, include artificial food dyes and conventional food or food colorants, such as ribonavin and b-carotene, caramel colorant, and also fruit, vegetable, and/or plant extracts such as grape, black currant, aronia, carrot, beetroot, red cabbage, and hibiscus, etc. or mixture thereof. The amount of coloring agent used will vary, depending on the agents used and the intensity desired in the finished product, preferably, from 0.0001% to 0.5%, by weight of the composition.

Examples of aromatizators, which can be used in the beverages of the present invention as a physiologically acceptable component, include natural cola-aromatizator, tea- aromatizator, etc. or mixture thereof.

Examples of food acid, which can be used in the beverages of the present invention as a physiologically acceptable component include siccine acid, malic acid, tartaric acid, gluconic acid, citric acid, lactic acid, maleic acid, fumaric acid, ascorbic acid, phosphoric acid, etc. or mixture thereof.

Example of bracer, which can be used in the beverages of the present invention as a physiologically acceptable component, include natural bracers, such as coffee, tea, kola nut, cacao pod, mate, royal jelly, yaupon, guarana paste, and yoco or synthetically produced caffeine, theobromine, and theophylline, etc. or mixture thereof. The amount of bracers in the compositions of the present invention can vary. Compositions of the present invention can comprise from 0.0005% to 1% of a bracer.

Examples of technological additives, which can be used in the beverages of the present invention, include preservative, emulsifier, acidulant, gelling agent, thickener, stabilizer, carbonate component, etc. or mixture thereof.

Examples of preservatives include polyphosphate preservatives (for example, sodium hexametapolyphosphate), sorbic acid, benzoic acid, and salts thereof, including calcium sorbate, sodium sorbate, potassium sorbate, calcium benzoate, sodium benzoate, potassium benzoate, EDTA (ethylenediaminetetraacetic acid), etc. or mixture thereof. The preservative is preferably included at levels from 0.0005% to 0.5%, by weight of the beverage composition. Wherein the beverage comprises a mixture of one or more preservatives, the total concentration of such preservatives is preferably maintained within these ranges.

Examples of emulsifier include gum acacia, modified food starches (e.g., alkenylsuccinate modified food starches), anionic polymers derived from cellulose (e.g., carboxymethylcellulose), gum ghatti, modified gum ghatti, xanthan gum, tragacanth gum, guar gum, locust bean gum, pectin, etc. and mixtures thereof.

Examples of acidulant include organic or inorganic edible acids. Organic acids which include citric acid, malic acid, fumaric acid, adipic acid, phosphoric acid, gluconic acid, tartaric acid, ascorbic acid, acetic acid, phosphoric acid or mixtures thereof. The acids can be present in their undissociated form or, alternatively, as their respective salts, for example, potassium or sodium hydrogen phosphate, potassium or sodium dihydrogen phosphate salts. Beverage acidity can be adjusted to and maintained within the requisite range by known and conventional methods, e.g., the use of one or more of the aforementioned acidulants. Typically, acidity within the above recited ranges is a balance between maximum acidity for microbial inhibition and optimum acidity for the desired beverage flavor. The acidulant can also serve as an antioxidant to stabilize beverage components. Examples of commonly used antioxidant include ascorbic acid, EDTA (ethylenediaminetetraacetic acid), and salts thereof.

Examples of thickener include suspending bodying agent, setting agent, gel builder, bulking agent, etc. or mixture thereof.

Examples of gelling agent include algenic acid, sodium alginate, potassium alginate, calcium alginate, agar etc. and mixtures thereof.

The same agents may also be used as gelling agent, food stabilizer, thickener. So, algenic acid and sodium alginate may be used not only as gelling agent but as stabilizer, thickener, emulsifier. Agar, which is gelling agent, may be used as thickener and emulsifier.

All physiologically acceptable components are used in forms, which are necessary for production one or another non- alcoholic beverage. Such components may be dispersed, solubilized, or otherwise mixed into the present compositions

For practicing the invention we offer the method of increasing ¹H₂¹⁶O content in non-alcoholic beverage in comparison with typical ¹H₂¹⁶O content in ordinary non- alcoholic beverages by addition highly pure water with content from 99.76 % to 99.99% of ¹H₂¹⁶O in necessary quantity to any non-alcoholic beverage composition.

Highly pure light water can be mixed with typical water with typical content of ¹H₂¹⁶O from 99.731% to 99.757 % by weight of water, for example typical drinking water. Obtained drinking water is enriched with ¹H₂¹⁶O, or in other words, light drinking water. Light drinking water of the invention can be table drinking water, mineralized water, mineral water, mineral table water, treatment-prophylactic mineral water, mineral-medicinal water, baby drinking water.

Herein and after, drinking water, mineralized water, mineral water, etc. with increased content of ¹H₂¹⁶O, in comparison with typical drinking, mineralized or mineral water can be named «light drinking water», «light mineralized water», «light mineral water», etc.

Examples of drinking water with typical content of ¹H₂¹⁶O from 99.731% to 99.757 % by weight of water, which can be used in the beverages of the present invention, include any bottled drinking water, artesian water and drinking water from other natural spring, etc.

Example of typical mineralized water which can be used in the beverages of the present invention, include artificial mineralized water, which is mixture of reverse osmosis water and necessary mineral components, etc.

Example of typical mineral water which can be used in the beverages of the present invention, include any water with ascertained mineral composition from natural mineral spring, etc.

In the present invention, the light drinking or mineral water enriched with ¹H₂¹⁶O can comprise inorganic salts and minerals in an amount up to necessary levels observed in typical drinking or mineral water. It can be prepared by mixing in certain ratios highly pure light water with typical drinking or mineral natural water with ascertained mineral composition. For example, the total mineralization of typical drinking water can be within the limits of 200-1000 mg/l, in mineral water the total mineralization can amount more than 10 g/l. Typically, in bottled drinking water the level of Ca amounts up to 130 mg/l, the level of Mg in bottled drinking water amounts up to 65 mg/l. The example of non-alcoholic beverage enriched with ¹H₂¹⁶O, which is drinking water, more specifically, table drinking water of the present invention, include table light drinking water enriched with H₂¹⁶O in include table light drinking waster enriched with ¹H₂¹⁶O in an amount up to 99.80% by weight with total mineralization 300 mg/l, with concentration of Ca 35 mg/l, Mg 10 mg/l.

Also, in the present invention, the light drinking or mineral water enriched with ¹H₂¹⁶O can be prepared by addition to highly pure light water of essential mineral components, such as inorganic salts and minerals, up to necessary level.

The examples of inorganic salt include sodium chloride, sodium bicarbonate, calcium chloride, magnesium sulfate, etc. or mixture thereof. The examples of mineral include Ca, Mg, Na, B, F, etc. or mixture thereof.

The light drinking water of the invention can be used for cooking and baby's formulae. Preferably, the light drinking water of the invention is bottled drinking water, and may be carbonated or still.

In case when highly pure light water is mixed with physiologically acceptable components (including typical water or without it), one can get any light non alcoholic beverage enriched with ¹H₂¹⁶O, comprising blended beverage, such as table beverage, beverage for special purpose, refreshing beverage, cool beverage, tonic, lemonade, non-alcoholic cocktail, and also juice, nectar, kissel, mors, tea, kvass, non-alcoholic beer.

Choice of acceptable component and final content of any physiologically acceptable component depends on the subsequent usage of any non-alcoholic beverage enriched with ¹H₂¹⁶O as table beverage, beverage for special purposes, refreshing beverage, cool beverage, tonic, lemonade, nonalcoholic cocktail, etc.

The examples of a non-alcoholic beverage for special purposes include beverage for fitness, sports drink, nutritive drink, etc.

In the present invention some of non-alcoholic beverage enriched with ¹H₂¹⁶O can be prepared by ordinary mixing the ingredients, i.e. by addition of necessary physiologically acceptable components to highly pure light water according to conventional methods. Obtained beverages enriched with ¹H₂¹⁶O are light beverages, for example blended beverage, such as lemonade, comprising highly pure light water can be name light lemonade. Light juice or nectar of the invention, can also be prepared by mixing highly pure light water with other necessary components. Examples in this case include mixing of the highly pure light water with natural juice or nectar, or with concentrated juice.

The other beverages comprising kissel, mors, tea, kvass, non-alcoholic beer can be prepared by usage of highly pure light water directly at any stage of non-alcoholic beverage production. Example in this case is non-alcoholic beer, when highly pure light water can be added at the stage of wort production.

Moreover, typical water may be substituted for highly pure light water enriched with ¹H₂¹⁶O in any non-alcoholic beverage. Blended beverage, kissel, mors, tea, kvass enriched with ¹H₂¹⁶O, etc. may be produced by this manner. Except water, other components of any non-alcoholic beverage enriched with ¹H₂¹⁶O remain the same according to the typical composition for conventional production methods of any blended beverage, kissel, mors, tea, kvass, non-alcoholic, etc. The solid ingredients may be dissolved in light water or in hot light water if required prior to addition to the other components.

Preferably, the non-alcoholic beverage is bottled, packaged or canned and saturated with carbon dioxide or still. Any conventional carbonation methodology may be utilized to make carbonated beverage compositions of this invention. The amount of carbon dioxide introduced into the beverage will depend upon the particular flavor system utilized and the amount of carbonation desired. The carbonated beverage can be placed into a container, such as a bottle or can, and then sealed.

Preservatives may or may not be needed for use in the present compositions. Techniques such as aseptic and/or clean-fill processing may be utilized to avoid preservatives. Typically drinks are pasteurized prior to filling in bottles, cans or other packs or are "in-pack pasteurized" after filling.

Water is an essential component of all biological systems. The functions of water are not limited to the role of the medium where the biochemical processes and diffusion of the metabolites take place. Water takes direct part in chemical reactions, in the osmoregulation and the transport of nutrients, structurization and stabilization of biopolymer molecules and permolecular systems providing their conformational mobility.

As mentioned above, light and heavy water isotopologues have distinct properties in biological system. For example, heavy water isotopologues decrease the rate of biochemical reactions, disturb conformational mobility of the molecules of biopolymers (Chervenak et al. JACS, 1994, 116 (23): 10533-10539: Makhatadze et al., Nature Struct. Biol., 1995, 2 (10): 852-855. Connelly et, al., PNAS, 1994, 91: 1964-1968. Cupane et al., Nucleic Acids Res. 1980, 8 (18): 4283-4303). Thus, heavy isotopes-comprising molecules in mammal organism decrease of functional resources of the organism.

As mention above, the content of isotope varieties of water molecule such as: ¹H₂¹⁶O, ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O in human organism directly depends on the content of said isotope varieties of water molecule in a non-alcoholic beverage used by human.

Usage of non-alcoholic beverage enriched with ¹H₂¹⁶O in an amount no less than 99.76 % by weight of water, prepared in accordance with present invention, is able to increase the content of light water molecules of ¹H₂¹⁶O and to decrease the content of ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O in human organism, and leads to improving of human wellness and life quality.

Non-alcoholic beverages enriched with ¹H₂¹⁶O in an amount no less than 99.76 % by weight of water of non-alcoholic beverage, which are obtained by the present invention, are a new product which has not been produced up to this time.

Non-alcoholic beverages enriched with ¹H₂¹⁶O in an amount no less than 99.76 % by weight of water of non-alcoholic beverage enriched with ¹H₂¹⁶O in an amount no less than 99.76 % by weight of water, are beverages with best quality, because they offer all advantages of light water.

The following examples are presented to demonstrate the invention.

### Example 1

This example, demonstrates the method for producing highly pure light water of the invention.

Highly pure light water comprising 99.99% of light isotopologue ¹H₂¹⁶O is prepared by distillation of typical natural water comprising 99.70% of light isotopologue ¹H₂¹⁶O with using the apparatus in FIG.1 under temperature 60°C and pressure 0.2 bars. The process of the distillation includes:
- evaporating typical natural water comprising 99.70% [C₁] of light isotopologue ¹H₂¹⁶O in boiling (see Fig 1,1) to produce water vapor;
- supplying the water vapor to the bottom (2) of distillation column (3);
- carrying out vapor-liquid contact between a descending liquid and an ascending vapor mainly on the surface of the contact device (4) (e.g. structured or random packing) within the distillation column, simultaneously the liquid and the vapor flow in mutually opposite directions over the surface of the contact device along a main flow direction which is a direction of the column axis;
- condensing water vapor with concentration of light isotopologue ¹H₂¹⁶O 99.99% [C₂] on condenser (5) installed on upper bound of the distillation column;
- and collecting a part of condensate as condensed highly pure light water comprising 99.99% of light isotopologue ¹H₂¹⁶O [C₂ > C₁] appropriate for producing light non-alcoholic beverage enriched with ¹H₂¹⁶O.

Then, the highly pure light water comprising 99.99% of light isotopologue ¹H₂¹⁶O is used for manufacturing of light non-alcoholic beverage enriched with ¹H₂¹⁶O no less than 99.76% by weight of water of said non-alcoholic beverages.

### Example 2

This example demonstrates non-alcoholic beverage, which is light mineralized drinking water enriched with ¹H₂¹⁶O.

| Ingredients | Content, weight % |
|---|---|
| Highly pure light water enriched with ¹H₂¹⁶O, wherein the content of ¹H₂¹⁶O is 99.80% by weight of water | 99.953 |
| Calcium chloride | 0.015 |
| Magnesium chloride | 0.007 |
| Sodium bicarbonate | 0.025 |
| Obtained light drinking water enriched with ¹H₂¹⁶O, wherein the content of ¹H₂¹⁶O is 99.80% by weight of water of said non-alcoholic beverage, total mineralization 0.047% or 470 mg/dm³ | 100 |

The method for preparing the light mineralized drinking water described in Example 2 was as follows: components such as bicarbonate and chloride salts of calcium, magnesium, and sodium were dissolved in light water at room temperature. Addition of certain inorganic salts to light water provides the necessary mineralization of light drinking water.

### Example 3

This example demonstrates non-alcoholic beverage, which is light mineral drinking water enriched with ¹H₂¹⁶O, obtained by mixing light water and mineral natural water with ascertained mineral composition.

| One of mineral compositions of natural mineral water: | mg/dm³ |
|---|---|
| Total mineralization | 2800-5300 |
| Cl (Chloride) | 1400-2500 |
| Na (Natrium) | 750-1540 |
| Ca (Calcium) | 170-340 |
| SO₄ (Sulphate) | 350-530 |
| HCO₃ (Hydrocarbonat) | 190 |
| H₃ BO₃ (Boric acid) | 40-43 |

### Light mineral drinking water enriched with ¹H₂¹⁶O

| Ingredients | Content, weight % |
|---|---|
| | |
| Distilled highly pure light water enriched with ¹H₂¹⁶O, wherein the content of ¹H₂¹⁶O is 99.90% by weight of water | 20 |
| Typical natural mineral water with typical content of ¹H₂¹⁶O in an amount 99.731% by weight of water and total mineralization 5.0 g/dm³ | 80 |
| Obtained light mineral drinking water enriched with ¹H₂O, wherein the content of ¹H₂¹⁶O is 99.7648% by weight of water of said non-alcoholic beverage, total mineralization about 4 g/dm³ | 100 |

Thus, the final content of ¹H₂¹⁶O in the light mineral drinking water amounts 99.7648%, that is higher than typical values in typical natural mineral waters.

The light mineral drinking water enriched with ¹H₂¹⁶O retains all natural inorganic ingredients of typical natural mineral water. As a result, it has sufficiently high biological effectiveness, savoury taste and all side benefits of light water.

### Example 4

This example demonstrates the non-alcoholic beverage composition enriched with ¹H₂¹⁶O, which is light non-alcoholic beverage.

| Ingredients | Content, weight percent |
|---|---|
| Glucose | 4 |
| Sucrose | 5.86 |
| Maltodextrin | 2.0 |
| Fruit Juice | 10 |
| Green Tea Extract | 0.12 |
| Guarana Extract | 0.06 |
| Ascorbic Acid | 0.04 |
| Arabinogalactan (CLEARTRAC, commercially available from Larex, Inc., St. Paul, MN) | 1.1 |
| Sodium Citrate | 0.1 |
| Citric Acid | 0.2 |
| Highly pure light water enriched with ¹H₂¹⁶O, wherein the content of ¹H₂¹⁶O is 99.80% by weight of water | up to 100 % |

The final content of ¹H₂¹⁶O in the water of the light non-alcoholic beverage amounts no less than 99.789 % under the most exacting requirement, that the total quantity of water of said beverage comprises up to 15 % typical water with the least content of ¹H₂¹⁶O 99.731%, which is in a part of solid and liquid ingredients, and suitable quantity (no less than 85 %) of light water with content of ¹H₂¹⁶O 99.80%.

The beverage composition is prepared by blending the listed components in a conventional manner.The light beverage enriched with ¹H₂¹⁶O has savoury taste, aroma and all additional benefits of the light water.

### Example 5

This example demonstrates the non-alcoholic beverage composition enriched with ¹H₂¹⁶O, which is light non-alcoholic beverage.

| Ingredients | Content, weight, g |
|---|---|
| Sugar | 124 |
| Food Grade Acids | 8.1 |
| Buffer Salts | 3.2 |
| Vitamins | 0.7 |
| Artificial Colors | 0.04 |
| Clouding Agent | 3.1 |
| Thickening Agent | 1.4 |
| Mandarin Flavor Emulsion | 1.76 |
| Highly pure light water enriched with ¹H₂¹⁶O, wherein the content of ¹H₂¹⁶O is 99.80% by weight of water | 910 |

The final content of ¹H₂¹⁶O in the water of light non-alcoholic beverage amounts no less than 99.792 % under the most exacting requirement, that quantity of typical water with the least content of ¹H₂¹⁶O 99.731 % amounts up to 12 % by weight of total beverage water.

Preparation: add ingredients to water with agitation. Stir till dissolved. Heat to 185.degree. F. 15 seconds. Pack aseptically.

The beverage enriched with ¹H₂¹⁶O, comprising light water, is better in sweet/sour blending, higher in aroma, and less bitter and sour in aftertaste, as compared with beverage, comprising typical water.

### Example 6

This example demonstrates the non-alcoholic nutritive beverage enriched with ¹H₂¹⁶O, which is light nutritive beverage.

| Ingredients | Content, Weight, g |
|---|---|
| Fructose-Glucose-Liquid Sugar | 150 |
| Purified honey | 2.0 |
| Guarana extract | 1.0 |
| Korean ginseng extract | 0.1 |
| Royal jelly | 0.05 |
| Vitamin C | 0.5 |
| Nicotinamide | 0.1 |
| Vitamin B.sub.1 hydrochloride | 0.02 |
| Vitamin B.sub.6 hydrochloride | 0.02 |
| L-Phenylalanine | 0.04 |
| L-Isoleucine | 0.01 |
| Citric acid | 1.5 |
| Perfume | 2.0 |
| Deionized highly pure light water enriched with ¹H₂¹⁶O, wherein the content of ¹H₂¹⁶O is 99.80% by weight of water | balance |
| Total | 1000 |

The final content of ¹H₂¹⁶O in the water of light non-alcoholic beverage amounts no less than 99.79 % under the most exacting requirement, that quantity of typical water with the least content of ¹H₂¹⁶O 99.731 % amounts up to 14 % by weight of total beverage water.

The beverage composition is prepared by blending the listed components in a conventional manner. The light nutritive beverage enriched with ¹H₂¹⁶O has all additional benefits of light water.

### Example 7

This example demonstrates the non-alcoholic beverage enriched with ¹H₂¹⁶O, which is light lemonade.

| Ingredients | Content, weight percent |
|---|---|
| Highly pure light water enriched with ¹H₂¹⁶O, wherein the content of ¹H₂¹⁶O is 99.79 % by weight of water | 86.97 |
| Sugar | 12 |
| Citric acid | 0.1 |
| Carbon dioxide | 0.6 |
| Natural aromatizator composition | 0.18 |
| Caramel colorant | 0.1 |
| Sodium benzoate | 0.05 |

The final content of ¹H₂¹⁶O in the water of light non-alcoholic beverage amounts no less than 99.78 % under the most exacting requirement, that quantity of typical water with the least content of ¹H₂¹⁶O 99.731 % amounts up to 12 % by weight of total beverage water.

The light lemonade enriched with ¹H₂¹⁶O has savoury taste, aroma and all additional benefits of light water.

### Example 8

This example demonstrates the light non-alcoholic beverage enriched with ¹H₂¹⁵O, which is light juice beverage.

| Ingredients | Content, weight % |
|---|---|
| Highly pure light water enriched with ¹H₂¹⁶O, wherein the content of 1H₂¹⁶O is 99.85 % by weight of water | 60 |
| Sugar (the content of typical water is 0.2%) | 20 |
| Concentrated orange (the content of typical water is 5 %) | 20 |
| | 100 |

The final content of ¹H₂¹⁶O in the water of light juice beverage amounts about 99.84 % by weight of water of non- alcoholic beverage.

The light juice beverage enriched with ¹H₂¹⁶O prepared by mixing light water with concentrated orange juice. The beverage has sufficiently high restorative properties.

### Example 9

This example demonstrates the method for producing non-alcoholic beer enriched with ¹H₂¹⁶O.

The non-alcoholic beer enriched with ¹H₂¹⁶O is prepared by conventional manner including the steps of:
(1) preparing a composition including a malt, molasses and highly pure light water with content ¹H₂¹⁶O = 99.88% in an amount not less than 90 % by weight of the composition
(2) boiling said composition to make a wort;
(3) adding a predetermined quantity of hops to said wort;
(4) adding a predetermined quantity of brewers' yeast to said wort;
(5) aerating said wort to secure yeast growth;
(6) pitching said wort with a yeast slurry to provide a cell count of about 80 to about 180 million yeast cells per ml;
(7) fermenting the pitched wort at a temperature of 3 to 10.degree. C. for 1 to 40 hours;
(8) removing the yeast from the fermented wort;
(9) filtering the resulting brew
(10) electrolyzing the resultant brew by passing said resultant brew past a pair of two copper electrodes
(11) adjusting the alcohol concentration of the brew with carbonated highly pure light water with content ¹H₂¹⁶O = 99.88% to a preselected level, and then aging the resultant brew for at least 24 hours.

The final content of ¹H₂¹⁶O in the total quantity of water into light non-alcoholic beer composition amounts no less than 99.82 % under the most exacting requirement, that the total quantity of water of said beverage comprises up to 40 % typical water with the least content of ¹H₂¹⁶O 99.731 %, which is received from solid and liquid beer ingredients, and suitable quantity (no less than 60 %) of light water with content of ¹H₂¹⁶O 99.88%.

Obtained non-alcoholic beer enriched with ¹H₂¹⁶O comprising the highly pure light water and not more than 3 % of alcohol in the aqueous-alcoholic composition has better consumer properties as stores all additional benefits of light water, has savoury taste and aroma.

### Example 10

30 adults consisting of men and women sampled light drinking water enriched with ¹H₂¹⁶O prepared in accordance with example 2, wherein the content of ¹H₂¹⁶O amounts 99.82 % by weight of water and total mineralization is 470 mg/dm³ in an amount from three to five glasses per day during one week.
Taste:
   Tasted nice - 27 persons,
   No taste - 3 persons.
Smell:
   Do not mind - 30 persons,
   Do mind - 0 person.

From these results, it can be seen that the light drinking water enriched with ¹H₂¹⁶O obtained according to the present invention is the beverage which is easy to drink for many people.

Moreover, during this week all volunteers who sampled the light drinking water registered a moderate diuretic effect and better digestive functions.

Furthermore, during the following month after weekly taking of light drinking water all volunteers registered at least one effect on health-related improvements such as recovering from fatigue, physical conditions and physical activity improved, endurance and stamina improved

This is evidence taking light drinking water enriched with ¹H₂¹⁶O prepared in accordance with present invention improves human wellness and life quality.

## Claims

1. A non-alcoholic beverage enriched with ¹H₂¹⁶O, wherein the content of ¹H₂¹⁶O is no less than 99.76% by weight of water of said non-alcoholic beverage, comprising:
A) a highly pure light water in an amount from 20% to 99.9%. by weight of said non-alcoholic beverage, wherein the highly pure light water is a composition comprising from 99:76 % to 99:99% of ¹H₂¹⁶O and residual amounts of ¹H₂¹⁷O,¹H₂¹⁸O, ¹H²H¹⁶O, ¹H²H¹⁷O, ¹H²H¹⁸O, ¹H₂¹⁶O, ²H₂¹⁷O, ²H₂¹⁸O up to 100% correspondingly;
B) a typical water with typical content of ¹H₂¹⁶O in an amount from 0 % to 80% by weight, of said non-alcoholic beverage, wherein said water is a composition comprising from 99.731 % to 99:757 % of ¹H₂¹⁶O and residual amounts of ¹H₂¹⁷O, ¹H₂¹⁸O, ¹H²H¹⁶O, ¹H²H¹⁷O, ¹H²H¹⁸0, ²H₂¹⁶O, ²H₂¹⁷_{O}, ²H₂¹⁸O up to 100% correspondingly; and
C) a physiologically acceptable component in an amount up to 100% by weight of said non-alcoholic beverage.

2. The non-alcoholic beverage of claim 1, wherein the content of ¹H₂¹⁶O is no less than 99:80 % by weight of water of said non-alcoholic beverage, comprising:
A) a highly pure light water in all amount from 20% to 99.9% by weight of said non-alcoholic beverage, wherein the highly pure light water is a composition comprising from 99.80 % to 99.99% of ¹H₂¹⁶O and residual amounts of ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O up to 100% correspondingly;
B) a typical water with typical content of ¹H₂¹⁶ O in an amount from 0% to 80% by weight of said non-alcoholic beverage, wherein said water is a composition comprising: from 99.731 % to 99.757 % of ¹H₂ ¹⁶O and residual amounts of ¹H₂¹⁷O, ¹H₂¹⁸_{O}, ¹H₂¹⁸O, ¹H²H¹⁶O_{,} ¹H₂H¹⁷O, ¹H²H¹⁸O, ²H₂¹⁶O, ²H₂¹⁷O, ²H₂¹⁸O up to 100% correspondingly; and
C) a physiologically acceptable component man amount up to 100% by weight of said non-alcoholic beverage.

3. The non-alcoholic beverage of claims 1-2, wherein the highly pure light water is selected from the group comprising distilled water, deionized water, reverse osmosis water.

4. The non-alcoholic beverage of claims 1-2, wherein the typical water with typical content of ¹H₂ ¹⁶O is selected from the group comprising drinking water, mineralized water, mineral water, distilled water, deionized water, reverse osmosis water.

5. The non-alcoholic beverage of claims 1-2, where the physiologically acceptable component is at least one selected from the group comprising natural fruit juice, vegetable juice, berry juice, nectar, dry beverage composition, dietary fiber, food component.

6. The non-alcoholic beverage of claims 1-2, wherein the physiologically acceptable component is at least one selected from the group comprising inorganic salt, mineral, nutrient, vitamin, flavanol, anti-oxidant, flavouring, extract, essence, colorant, aromatizator, food acid, bracer, technological additive.

7. The non-alcoholic beverage of claims 1-2, wherein said non-alcoholic beverage is a drinking water.

8. The non-alcoholic beverage of claim 1-2 wherein said non-alcoholic
beverage is selected from the group comprising table drinking water, mineralized water mineral water, mineral table water, treatment-prophylactic
mineral water, mineral-medicinal water, baby drinking water.

9. The non-alcoholic beverage of claims 7-8, wherein it is saturated with carbon dioxide.

10. The non-alcoholic beverage of beverage is a blended beverage.

11. The non-alcoholic beverage of claim 10, wherein said non-alcoholic beverage is selected from the group comprising table beverage, beverage for special purpose, refreshing beverage, cool beverage, tonic, lemonade, non-alcoholic cocktail.

12. The non-alcoholic beverage of claims 10-11, wherein it is saturated with carbon dioxide.

13. The non-alcoholic beverage of claims 1-2, wherein said non-alcoholic
beverage is selected from the group comprising juice, nectar, kissel, mors, tea, kvass, non-alcoholic beer.

14. The non-alcoholic beverage of claim 13, wherein it is saturated with carbon dioxide.

## Patentansprüche

1. Mit ¹H₂¹⁶O angereichertes, alkoholfreies Getränk, wobei der Gehalt an ¹H₂¹⁶O nicht weniger als 99,76 Gew.-% des Wassers des alkoholfreien Getränks ausmacht, das aufweist:
A) hochreines leichtes Wasser in einer Menge von 20 bis 99,9 Gew.-% des alkoholfreien Getränks, wobei das hochreine leichte Wasser eine Zusammensetzung ist, die 99,76 bis 99,99% ¹H₂¹⁶O und auf 100% Restmengen von ¹H₂¹⁷O, ¹H₂¹⁸O, ¹H²H¹⁶O, ¹H²H¹⁷O, ¹H²H¹⁸O, ²H₂¹⁶O, ²H₂¹⁷O, 2H₂¹⁸O aufweist;
B) typisches Wasser mit einem typischen ¹H₂¹⁶O-Gehalt in einer Menge von 0 bis 80 Gew.-% des alkoholfreien Getränks, wobei dieses Wasser eine Zusammensetzung ist, die 99,731 bis 99,757% ¹H₂¹⁶O und auf 100% Restmengen von ¹H₂¹⁷O, ¹H₂¹⁸O, ¹H²H¹⁶O, ¹H₂H¹⁷O, ¹H²H¹⁸O, 2H₂¹⁶O, 2H₂¹⁷O, ²H₂¹⁸O aufweist; und
C) eine physiologisch akzeptable Komponente in einer Menge auf 100 Gew.-% des alkoholfreien Getränks.

2. Alkoholfreies Getränk nach Anspruch 1, wobei der Gehalt an ¹H₂¹⁶O nicht weniger als 99,80 Gew.-% des Wassers des alkoholfreien Getränks ausmacht, das aufweist:
A) hochreines leichtes Wasser in einer Menge von 20 bis 99,9 Gew.-% des alkoholfreien Getränks, wobei das hochreine leichte Wasser eine Zusammensetzung ist, die 99,80 bis 99,99% ¹H₂¹⁶O und auf 100% Restmengen von ¹H₂¹⁷O, ¹H₂¹⁸O, ¹H²H¹⁶O, ¹H²H¹⁷O, ¹H²H¹⁸O, ²H₂¹⁶O, ²H2¹⁷O, 2H₂¹⁸O aufweist;
B) typisches Wasser mit einem typischen ¹H₂¹⁶O-Gehalt in einer Menge von 0 bis 80 Gew.-% des alkoholfreien Getränks, wobei dieses Wasser eine Zusammensetzung ist, die 99,731 bis 99,757% ¹H₂¹⁶O und auf 100% Restmengen von ¹H₂¹⁷O, ¹H₂¹⁸O, ¹H²H¹⁶O ¹H²H¹⁷O, ¹H²H¹⁸O, ²H₂¹⁶O, 2H₂¹⁷O, 2H₂¹⁸O aufweist; und
C) eine physiologisch akzeptable Komponente in einer Menge auf 100 Gew.-% des alkoholfreien Getränks.

3. Alkoholfreies Getränk nach den Ansprüchen 1 bis 2, wobei das hochreine leichte Wasser unter destilliertem Wasser, deionisiertem Wasser und Umkehrosmose-Wasser ausgewählt ist.

4. Alkoholfreies Getränk nach den Ansprüchen 1 bis 2, wobei das typische Wasser mit einem typischen ¹H₂¹⁶O-Gehalt unter Trinkwasser, mineralisiertem Wasser, Mineralwasser, destilliertem Wasser, deionisiertem Wasser und Umkehrosmose-Wasser ausgewählt ist.

5. Alkoholfreies Getränk nach den Ansprüchen 1 bis 2, wobei die physiologisch akzeptable Komponente mindestens eine Komponente ist, die unter natürlichem Fruchtsaft, Gemüsesaft, Beerensaft, Nektar, pulverförmigen Getränkezusammensetzungen, Ballaststoffen, Lebensmittelkomponenten ausgewählt ist.

6. Alkoholfreies Getränk nach den Ansprüchen 1 bis 2, wobei die physiologisch akzeptable Komponente mindestens eine Komponente ist, die unter anorganischen Salzen, Mineralstoffen, Nährstoffen, Vitaminen, Flavanolen, Antioxidantien, Aromastoffen, Extrakten, Essenzen, Farbstoffen, aromatisierenden Stoffen, Lebensmittelsäuren, Stärkungsmitteln, technischen Additiven ausgewählt ist.

7. Alkoholfreies Getränk nach den Ansprüchen 1 bis 2, wobei es sich bei dem alkoholfreien Getränk um ein Trinkwasser handelt.

8. Alkoholfreies Getränk nach den Ansprüchen 1 bis 2, wobei das alkoholfreie Getränk unter Tafelwasser, mineralisiertem Wasser, Mineralwasser, Mineraltafelwasser, Mineralwasser zur prophylaktischen Behandlung, Heilmineralwasser und Baby-Trinkwasser ausgewählt ist.

9. Alkoholfreies Getränk nach den Ansprüchen 7 bis 8, wobei es mit Kohlendioxid gesättigt ist.

10. Alkoholfreies Getränk nach den Ansprüchen 1 bis 2, wobei das alkoholfreie Getränk ein Mischgetränk ist.

11. Alkoholfreies Getränk nach Anspruch 10, wobei das alkoholfreie Getränk unter Tischgetränken, Getränken für Spezialzwecke, Erfrischungsgetränken, Kaltgetränken, Tonic, Limonade, alkoholfreien Cocktails ausgewählt ist.

12. Alkoholfreies Getränk nach den Ansprüchen 10 bis 11, wobei es mit Kohlendioxid gesättigt ist.

13. Alkoholfreies Getränk nach den Ansprüchen 1 bis 2, wobei das alkoholfreie Getränk unter Saft, Nektar, Kissel, Mors, Tee, Kwas, alkoholfreiem Bier ausgewählt ist.

14. Alkoholfreies Getränk nach Anspruch 13, wobei es mit Kohlendioxid gesättigt ist.

## Revendications

1. Boisson non alcoolisée enrichie en ¹H₂¹⁶O, dans laquelle la teneur en ¹H₂¹⁶O n'est pas inférieure à 99,76 % en poids de l'eau de ladite boisson non alcoolisée, comprenant :
A) une eau légère très pure en une quantité de 20 % à 99,9 % en poids de ladite boisson non alcoolisée, l'eau légère très pure étant une composition comprenant de 99,76 % à 99,99 % de ¹H₂¹⁶O et des quantités résiduelles de ¹H₂¹⁷O, ¹H₂¹⁸O, ¹H²H¹⁶O, ¹H²H¹⁷O, ¹H²H¹⁸O, ²H₂¹⁶O, ²H₂¹⁷O,²H₂¹⁸O allant jusqu'à 100 % proportionnellement ;
B) une eau typique ayant une teneur typique en ¹H₂¹⁶O en une quantité de 0 % à 80 % en poids de ladite boisson non alcoolisée, ladite eau étant une composition comprenant de 99,731 % à 99,757 % de ¹H₂¹⁶O et des quantités résiduelles de ¹H₂¹⁷O, ¹H₂¹⁸O, ¹H²H¹⁶O, ¹H²H¹⁷O, ¹H²H¹⁸O, 2H₂¹⁶O, ²H₂¹⁷O, 2H₂¹⁸O allant jusqu'à 100 % proportionnellement ; et
C) un composant physiologiquement acceptable en une quantité allant jusqu'à 100 % en poids de ladite boisson non alcoolisée.

2. Boisson non alcoolisée selon la revendication 1, dans laquelle la teneur en ¹H₂¹⁶O n'est pas inférieure à 99,80 % en poids de l'eau de ladite boisson non alcoolisée, comprenant :
A) une eau légère très pure en une quantité de 20 % à 99,9 % en poids de ladite boisson non alcoolisée, l'eau légère très pure étant une composition comprenant de 99,80 % à 99,99 % de ¹H₂¹⁶O et des quantités résiduelles de ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O allant jusqu'à 100 % proportionnellement ;
B) une eau typique ayant une teneur typique en ¹H₂¹⁶O en une quantité de 0 % à 80 % en poids de ladite boisson non alcoolisée, ladite eau étant une composition comprenant de 99, 731 % à 99, 757 % de ¹H₂¹⁶O et des quantités résiduelles de ¹H₂¹⁷O, ¹H₂¹⁸O, ¹H²H¹⁶O, ¹H²H¹⁷O, ¹H²H¹⁸O, ²H₂¹⁶O_{,} ²H₂¹⁷O, ²H₂¹⁸O allant jusqu'à 100 % proportionnellement ; et
C) un composant physiologiquement acceptable en une quantité allant jusqu'à 100 % en poids de ladite boisson non alcoolisée.

3. Boisson non alcoolisée selon la revendication 1 ou 2, dans laquelle l'eau légère très pure est choisie dans le groupe constitué par une eau distillée, une eau désionisée, une eau obtenue par osmose inverse.

4. Boisson non alcoolisée selon la revendication 1 ou 2, dans laquelle l'eau typique ayant une teneur typique en ¹H₂¹⁶O est choisie dans le groupe comprenant une eau potable, une eau minéralisée, une eau minérale, une eau distillée, une eau désionisée, une eau obtenue par osmose inverse.

5. Boisson non alcoolisée selon la revendication 1 ou 2, dans laquelle le composant physiologiquement acceptable est au moins choisi dans le groupe comprenant un jus de fruit naturel, un jus de légume, un jus de baie, un nectar, une composition pour boisson déshydratée, une fibre alimentaire, un composant alimentaire.

6. Boisson non alcoolisée selon la revendication 1 ou 2, dans laquelle le composant physiologiquement acceptable est au moins choisi dans le groupe comprenant un sel inorganique, un minéral, un nutriment, une vitamine, un flavanol, un antioxydant, un agent aromatisant, un extrait, une essence, un colorant, un agent aromatisant, un acide alimentaire, un tonique, un additif technologique.

7. Boisson non alcoolisée selon la revendication 1 ou 2, ladite boisson non alcoolisée étant une eau potable.

8. Boisson non alcoolisée selon la revendication 1 ou 2, ladite boisson non alcoolisée étant choisie dans le groupe comprenant une eau potable de table, une eau minéralisée, une eau minérale, une eau minérale de table, une eau minérale obtenue par traitement prophylactique, une eau minérale médicinale, une eau potable pour bébé.

9. Boisson non alcoolisée selon la revendication 7 ou 8, qui est saturée avec du dioxyde de carbone.

10. Boisson non alcoolisée selon la revendication 1 ou 2, ladite boisson non alcoolisée étant une boisson mixée.

11. Boisson non alcoolisée selon la revendication 10, ladite boisson non alcoolisée étant choisie dans le groupe comprenant une boisson de table, une boisson pour un usage spécifique, une boisson rafraîchissante, une boisson froide, un tonique, une limonade, un cocktail non alcoolisé.

12. Boisson non alcoolisée selon la revendication 10 ou 11, qui est saturée avec du dioxyde de carbone.

13. Boisson non alcoolisée selon la revendication 1 ou 2, ladite boisson non alcoolisée étant choisie dans le groupe comprenant un jus, un nectar, du kissel, du mors, un thé, du kvass, une bière non alcoolisée.

14. Boisson non alcoolisée selon la revendication 13, qui est saturée avec du dioxyde de carbone.
